# EUROPEAN PATENT APPLICATION

(11) **EP 1 946 760 A1**
(43) Date of publication of application: **23.07.2008**
(21) Application number: 06811588.0
(22) Date of filing: 11.10.2006
(51) Int. Cl.: A61K 31/702, A23L 1/30, A61P 1/00

(54) **COMPOSITION FOR IMPROVING INTESTINAL FLORA**

(30) Priority: 13.10.2005 JP 2005298252
(71) Applicant: Meiji Seika Kaisha, Ltd., Tokyo 104-8002 (JP)
(72) Inventor: MURASHIMA, Koichiro, Sakado-shi Saitama 350-0289 (JP); ENDO, Hiroya, Sakado-shi Saitama 350-0289 (JP); BABA, Yuko, Sakado-shi Saitama 350-0289 (JP); NAKAMURA, Hirofumi, Sakado-shi Saitama 350-0289 (JP)
(74) Representative: Ahner, Francis
(86) International application number: PCT/JP2006/320281
(87) International publication number: WO 2007/043563

(57) **Abstract**

A composition provided by the present invention for improving intestinal microflora includes 1-kestose as an active ingredient. The present invention further provides a lactic acid bacteria proliferating agent including 1-kestose as an active ingredient, which proliferates *Bifidobacterium* and lactic acid bacteria simultaneously. According to the present invention, intestinal microflora can be improved by growth of intestinal bacteria functioning beneficially to humans, especially both *Bifidobacterium* and lactic acid bacteria.

## Description

### CROSS-REFERENCE TO RELATED APPLICATION

The present patent application is based on and claims benefit of priority from prior Japanese Patent Application No. 2005-298252 filed on October 13, 2005, and the entirety of the disclosure thereof is incorporated herein by reference.

### BACKGROUND OF THE INVENTION

### Field of the Invention

The present invention relates to a composition for improving intestinal microflora of humans, *etc.* and a food and drink having such a function.

### Related Art

In human intestine, more than 100 bacteria live symbiotically and form a complex microflora. Among the bacteria forming such microflora, *Bifidobacterium* and lactic acid bacteria as represented by the genus *Lactobacillus* are known to act beneficially to humans. Therefore, it has been believed beneficial to the maintenance of human health to form intestinal microflora, where there is a lot of *Bifidobacterium* and lactic acid bacteria.

Regarding *Bifidobacterium,* substances, such as an oligosaccharides or lactic acid bacteria cells, have been found, which increase *Bifidobacterium* about 10-fold in human intestine.
While *Bifidobacterium* is a dominant bacteria populating 10⁹ to 10¹⁰ CFU/g in human intestinal microflora, the population of lactic acid bacteria is 10⁵ to 10⁶ CFU/g, which is about one 10,000th of *Bifidobacterium.* Therefore, it has been considered that it is difficult to allow growth of lactic acid bacteria in the intestine, and that the benefit of such growth would be limited.

However, owing to accumulation of knowledge about the activation effect on intestinal immunity of lactic acid bacteria, and the like (e.g. see Japanese Patent Laid-Open No. 1993-132425, Japanese Patent Laid-Open Publication No. 1995-228536 and WO 99/10476 A (corresponding to Japanese Patent Laid-Open Publication No. 2001-513990)), it has been becoming clear that the benefits of growth of lactic acid bacteria to humans can be larger than that considered in the past despite its small population rate in intestinal microflora.

Consequently, a substance, which can actually proliferate lactic acid bacteria in human intestine, has been desired. It is more appropriate to proliferate both *Bifidobacterium* and lactic acid bacteria to improve effectively human intestinal microflora. Therefore, a substance, which can proliferate simultaneously *Bifidobacterium* and lactic acid bacteria, has been also desired.

As for a substance to proliferate lactic acid bacteria, a substance which can proliferate lactic acid bacteria *in vitro* or in animal intestine has been found (e.g. see Japanese Patent No. 2823640, Japanese Patent Laid-Open Publication No. 1993-15366, Japanese Patent Laid-Open Publication No. 1994-125771 and Japanese Patent Laid-Open Publication No. 1995-267866). However, such a growth effect is limited to growth tests of intestinal bacteria in vitro or on a culture plate, and feeding tests to animals, and no case has been known to the present inventors, in which a substance is in-taken by humans, which actually proliferates lactic acid bacteria in human intestine.

While, 1-kestose is a trisaccharide, in which at the 1-position of fructose in a sucrose molecule another fructose is bonded by a β-2,1 linkage. A fructo-oligosaccharide is known to be less likely to decay teeth and not to be digested by intravital digestive enzymes, and to have a specific growth promoting effect on intestinal *Bifidobacterium.* 1-Kestose is a component of the fructo-oligosaccharide (see WO 97/021718 (Japanese Patent Publication No. 3459264)).

### SUMMARY OF THE INVENTION

The present inventors have now found that, by allowing a human to orally ingest 1-kestose, which *Bifidobacterium* and lactic acid bacteria can utilize but many of other intestinal bacteria can probably not utilize, and examining in detail microfloras in the feces, growth of lactic acid bacteria can be achieved by intake of 1-kestose in addition to growth of *Bifidobacterium.* The present inventors have further found that the effect by intake of 1-kestose is higher in humans with a count of lactic acid bacteria in the feces being a certain value or higher. The present invention is based on these findings.

An object of the present invention is to provide a composition, which can improve intestinal microflora by growth of intestinal bacteria functioning beneficially to humans, especially both *Bifidobacterium* and lactic acid bacteria.

A composition for improving intestinal microflora according to the present invention comprises 1-kestose as an active ingredient.

A lactic acid bacteria proliferating agent according to the present invention can proliferate simultaneously *Bifidobacterium* and lactic acid bacteria and comprises 1-kestose as an active ingredient.

A food and drink according to the present invention comprises the lactic acid bacteria proliferating agent according to the present invention.
In another aspect of the present invention, the food and drink according to the present invention includes 1-kestose in an amount provided in a range of 0.01 to 50 g per day per adult as the amount of 1-kestose, as well as viable cells of lactic acid bacteria.

In another aspect of the present invention, there is provided a method for improving intestinal microflora of mammals, said method comprising the step of administering an effective amount of 1-kestose to mammals or allowing mammals to ingest an effective amount of 1-kestose.

Further in another aspect of the present invention, theree is provided use of 1-kestose in the manufacture of a composition for improving intestinal microflora is provided.

According to the present invention, through an oral intake (ingestion) of an active ingredient of 1-kestose as a medicament or a food and drink by mammals including humans, the growth of intestinal bacteria functioning beneficially to the in-taking subject is induced to improve intestinal microflora. More particularly, through an oral intake by a human of the composition for improving intestinal microflora and the lactic acid bacteria proliferating agent according to the present invention, *Bifidobacterium* can be proliferated in human intestine, and at the same time lactic acid bacteria living symbiotically in the intestine or in-taken orally can be proliferated, and as the result a human intestinal microflora can be improved.

### BRIEF DESCRIPTION OF DRAWINGS

Figure 1 illustrates a result of Example 1 showing particularly the growth effect on lactic acid bacteria belonging to genus *Lactobacillus* in human feces by intake of the composition for improving intestinal microflora according to the present invention;
Figure 2 illustrates a result of Example 1 showing particularly the growth effect on *Bifidobacterium* in human feces by intake of the composition for improving intestinal microflora according to the present invention;
Figure 3 illustrates a result of Example 2 showing particularly the growth effect on lactic acid bacteria belonging to genus *Lactobacillus* in human feces by intake of the composition for improving intestinal microflora according to the present invention; and
Figure 4 illustrates a result of Example 2 showing particularly the growth effect on *Bifidobacterium* in human feces by intake of the composition for improving intestinal microflora according to the present invention.

### DETAILED DESCRIPTION OF THE INVENTION

### 1-Kestose

As described above, 1-kestose is an oligosaccharide classified as a trisaccharide, in which at the 1-position of fructose in a sucrose molecule another fructose is bonded by a β-2,1 linkage. For the use in the present invention, 1-kestose may be purchased from the market, or produced from certain prepared raw materials. There are no particular restrictions on a method for producing 1-kestose, and 1-kestose can be produced, for example, by a method described in WO 97/021718 (Japanese Patent Publication No. 3459264). If necessary, 1-kestose with the purity of 98% or higher can be obtained by the above method. Further, as 1-kestose, a commercially available mixture product containing 1-kestose, such as Meioligo P (Trade Name) (available by Meiji Seika Kaisha, Ltd.), which is a mixture of short chain fructo-oligosaccharides (with the components of: 1-kestose at 35% (by weight), nystose at 55%, and fructo-furanosyl nystose at 10%), may be used.

### Lactic acid bacteria

Any lactic acid bacteria may be used for the present invention insofar as it can inhabit as intestinal bacteria. Examples include lactic acid bacteria belonging to any of genus *Lactobacillus,* genus *Streptococcus,* genus *Leuconostoc,* genus *Pediococcus,* genus *Enterococcus* and genus *Lactococcus.* Commercially available lactic acid bacteria as well as the cultures of a sample from intestine *etc.* may be used.

Lactic acid bacteria are in a state of viable cells, in case used together with 1-kestose in the present invention. Here viable cells mean cells which reach live by oral intake to the intestine of the in-taking subject, such as a human, and various forms can be selected, for example, freeze-dried cells, wet cells or a culture fluid. For the use in the composition for improving intestinal microflora and the lactic acid bacteria proliferating agent according to the present invention, viable cells of lactic acid bacteria are preferably in a form of freeze-dried cells.

Preferable lactic acid bacteria in the present invention are lactic acid bacteria belonging to genus *Lactobacillus.* Specific examples of lactic acid bacteria belonging to genus *Lactobacillus* include: *Lactobacillus mucosae, Lactobacillus salivarius, Lactobacillus acidophilus, Lactobacillus lactis, Lactobacillus plantarum, Lactobacillus pentosus, Lactobacillus arizonensis, Lactobacillus rhamnosus, Lactobacillus brevis, Lactobacillus casei, Lactobacillus paracasei, Lactobacillus bulgaricus, Lactobacillus delbruckii, Lactobacillus arabinosus, Lactobacillus caucasicus, Lactobacillus leishmanni, Lactobacillus musicus, Lactobacillus themophilus, Lactobacillus fermentum and Lactobacillus helveticus.*

Specific examples of other genuses include: *Streptococcus themophilus, Streptococcus cremoris, Leuconostoc mesenteroides, Pediococcus pentosaceus, Enterococcus faecalis, Lactococcus lactis, Enterococcus hirae and Enterococcus faecium.*

Quantitative determination of intestinal lactic acid bacteria counts can be carried out by a conventional method. For example, the lactic acid bacteria counts can be quantitatively determined according to the Mitsuoka's method (Mitsuoka Tomotari, "Chounaikin no Sekai (The World of Intestinal Bacteria)", Sobunsha Co., Ltd., Tokyo, p. 53-65) by sampling feces of a tested subject. The intestinal *Bifidobacterium* count can be similarly quantitatively determined.

### Use

The active ingredient according to the present invention is effective in improving intestinal microflora. More specifically, the active ingredient according to the present invention is able to proliferate *Bifidobacterium* in the intestine of a subject in-taking the active ingredient and at the same time proliferate lactic acid bacteria inhabiting the intestine symbiotically or in-taken orally. As illustrated in Example below, the ingestion of 1-kestose in a human actually increased *Bifidobacterium* and *Lactobacillus sp.* (Example 1). It was further confirmed by fact that the 1-kestose contained in a composition to be in-taken at the concentration of about 35% was similarly effective (Example 2). According to the above, it has been demonstrated by the fact that 1-kestose can increase *Bifidobacterium* in intestine in an in-taking subject, as well as intestinal lactic acid bacteria. Accordingly the active ingredient in the present invention can, for a subject requiring the improvement of its intestinal microflora, induce growth of intestinal *Bifidobacterium* and lactic acid bacteria, proliferate intestinal bacteria functioning beneficially, and, as the result, exert improving effects on intestinal microflora. Further, since the active ingredient according to the present invention is a part of the material that has been consumed as food, its safety has been confirmed and it can be safely used for mammals (e.g. human, mouse, rat, rabbit, dog, cat, cattle, horse, pig, monkey; preferably for a human).

Further it has been confirmed that, in the present invention, the effect of the composition containing 1-kestose is stronger in a human whose lactic acid bacteria count in the feces is at a certain value (≥10⁶ CFU/g) (Example 1) or higher. Therefore, since intestinal microflora improving effect is more efficient if the intestinal lactic acid bacteria count is a certain value or higher, in the event that the intestinal lactic acid bacteria count is judged too low (for example, in the event that the lactic acid bacteria count in the feces is found too low), to the composition for improving intestinal microflora and the lactic acid bacteria proliferating agent according to the present invention, viable cells of lactic acid bacteria are further added, so that the improving effect of intestinal microflora is further increased by intake of the same.

In a preferable aspect of the present invention, the composition for improving intestinal microflora or the lactic acid bacteria proliferating agent according to the present invention further comprises viable cells of lactic acid bacteria.

In another preferable aspect of the present invention, as described above, there is provided a method for improving intestinal microflora of mammals, comprising the step of administering an effective amount of 1-kestose to mammals or allowing mammals to ingest an effective amount of 1-kestose. Here "an effective amount" means the minimum quantity of the active ingredient required for improving intestinal microflora.

In another preferable aspect, in the method for improving intestinal microflora, viable cells of lactic acid bacteria are administered or ingested together with 1-kestose. More preferably, the method according to the present invention comprises administering or feeding viable cells of lactic acid bacteria prior to or simultaneously with 1-kestose, so that the count of intestinal lactic acid bacteria in humans becomes 10⁶ CFU/g or more as measured in the feces.

In the present invention, "intestinal microflora" means a state of intestinal bacterial groups formed by beneficial bacteria, such as *Bifidobacterium* or lactic acid bacteria, harmful bacteria, such as *Clostridium perfringens,* and opportunistic bacteria not belonging to either of the two, inhabiting intestine (for example, from the end of the small intestine over the large intestine). It is known that a balance of intestinal microflora may be collapsed by an unbalanced diet, overeating, overdrinking, drug intake, stress, overwork, bacterial infection, disease or aging. It is further known that by a collapse of a balance of intestinal microflora, for example, harmful bacteria may grow to increase hazardous substances against health, causing constipation or diarrhea, deteriorating immunity, accelerating aging, and moreover causing cancer or other lifestyle-related diseases.

Therefore, in the present invention, "improvement of intestinal microflora" means maintenance of a balance of intestinal microflora, which is of value to maintain and improve human health, or recovery from an unbalanced intestinal microflora to an original condition. Further the improvement of intestinal microflora preferably means, according to the present invention, the improvement carried out by simultaneous proliferation of intestinal *Bifidobacterium* and lactic acid bacteria.

If a subject, who ingests the composition or the food and drink according to the present invention, is a human, the human may be healthy or sick. However, an especially remarkable effect is obtained on disordered bowel, such as constipation symptoms typical with a diabetic patient (Example). Therefore, according to a preferable aspect of the present invention, "improvement of intestinal microflora" may mean improvement of disordered bowel, more preferably improvement of disordered bowel such as constipation symptoms typical with a diabetic patient.

### Composition or food and drink

As described above, the composition according to the present invention includes 1-kestose as an active ingredient.

Here "comprising as an active ingredient" means naturally that it may of course include a physiologically acceptable carrier according to the desired product form, but also that it may include other adjuvant components that can be used together. Namely, the composition according to the present invention is prepared by using 1-kestose as an active ingredient, and, optionally, mixing a physiologically acceptable carrier or a diluent, *etc.* The composition according to the present invention can be mainly orally administered or fed, and provided in various forms, such as a medicament or a food and drink. Examples of forms for oral ingestion (administration) include: a food and drink, granules, powders, a tablet (including a sugar coated tablet), a pill, a capsule, a solution, syrup, an emulsion and a suspension. Such intake forms can be formulated according to a generally practiced method in the art together with a physiologically acceptable carrier. Examples of physiologically acceptable carriers include: an excipient, a binder, an additive, a flavor, a buffering agent, a thickener, a colorant, a stabilizer, an emulsifier, a dispersant, a suspending agent, a disintegrant, a lubricant and an antiseptic agent. More specific examples include: magnesium carbonate, magnesium stearate, talc, sugar, lactose, pectin, dextrin, starch, gelatin, tragacanth, methyl cellulose, sodium carboxymethyl cellulose and low meting point wax.

Here the meaning of "including/comprising as an active ingredient" can be similarly applied to the lactic acid bacteria proliferating agent. Consequently, the lactic acid bacteria proliferating agent according to the present invention can be used as a medicament or a food and drink. Further, the lactic acid bacteria proliferating agent may include the composition according to the present invention.

Examples of other adjuvant components to be used together include: a vitamin component (e.g. vitamin C, vitamin E, taurine), antibiotics, glycogen, amino acids, peptides and minerals (e.g. zinc, iron, cupper, manganese).

Among the aforementioned ingestion forms, a solid oral preparation, such as a tablet, can be prepared as follows. To an active ingredient are added, for example, an excipient (e.g. lactose, sucrose, starch, mannitol), a disintegrant (e.g. calcium carbonate, calcium carboxymethyl cellulose) and a binder (e.g. alpha-starch, gum Arabic, carboxymethyl cellulose, polyvinylpyrrolidone, hydroxypropyl cellulose), or a lubricant (e.g. talc, magnesium stearate, polyethyleneglycol), and the mixture is compression molded and, as required, coated for the purposes of taste masking, or enteric or sustaining delivery by a generally practiced method in the art, to an oral preparation. As a coating material ethyl cellulose, hydroxymethyl cellulose, polyoxyethyleneglycol, etc. can be used.

It is intended to direct the composition according to the present invention not only to pharmaceuticals but also to a food. Therefore the food and drink according to the present invention can include an effective amount of the active ingredient according to the present invention. Here "including/comprising an effective amount of the active ingredient" means: that the active ingredient is included in such as amount that by ingestion of an individual food and drink at the amount usually consumed, the active ingredient can exert its effect. The food and drink according to the present invention therefore includes foods and drinks: comprising the active ingredient according to the present invention added as it is; comprising the composition according to the present invention; comprising the lactic acid bacteria proliferating agent according to the present invention; and comprising 1-kestose in an amount provided in a range of 0.01 to 50 g per day per adult, as well as viable cells of lactic acid bacteria. Further, the food and drink according to the present invention may be a food and drink prepared by adding conventional additives, such as a stabilizer, to the active ingredient according to the present invention; a food and drink prepared by further adding various proteins, saccharides, fat, trace elements and vitamins to such a food and drink; a liquid, semi-liquid or solid food and drink; a paste-like food and drink; or general food added with the active ingredient.

In the present invention, "a food and drink" means what mammals can in-take, and is other than medicaments, without any other particular restrictions. It may be in any of a liquid, semi-liquid or solid form. Therefore a form of a beverage is included in the food and drink. The food and drink may be also in a tablet form as used for a nutritional supplement.

"A food and drink" in the present invention such categories as a health food, a functional food, a designated health food, a nutritional supplement, a food with a reduction of disease risk claim and a medical food. The term "a food and drink" may further include a feed in case of mammals other than humans. The abovementioned designated health food means a food under possible legal control in a relevant country from the standpoint of public health, as in the case a food is prepared or sold with an intention to maintain or improve intestinal microflora. Such a food can include a food bearing a label claiming the possibility of reduction of disease risk, namely a food with a reduction of disease risk claim. Here, a reduction of disease risk claim means labeling for a food having the possibility of reduction of disease risk, and such labeling may be defined or approved according to or referring to the regulations of the Joint FAO/WHO Food Standards Programme (CODEX Alimentarius Commission).

The active ingredient according to the present invention has an effect of improving intestinal microflora. Consequently, by mixing the active ingredient according to the present invention with a daily food, a health food, a functional food, a nutritional supplement (a food containing at least any one of minerals, such as calcium or magnesium, and vitamins, such as vitamin K, *etc.),* a food and drink with a function based on the aforementioned effect can be provided.

According to a preferable aspect of the present invention, a food and drink which is used for improving intestinal microflora is provided. According to a more preferable aspect, a food and drink labeled with a function of improving intestinal microflora is provided. Here, such labeling about function attached to a food and drink can be placed on any of a food itself, a container, a package, an instruction, an attached document and an advertisement.

Specific examples of the food and drink according to the present invention include: lactic products, such as milk, a lactic drink, ice cream, cheese, and yogurt; various beverages, such as juice, a soft drink, a functional drink and an alcoholic beverage; fermented products, such as miso, yogurt, lactic acid bacteria, a fermented milk beverage and pickles; foods containing carbohydrate, such as rice; paste products, such as fish paste; retort foods; soup; bean products; and various confectioneries such as western-style confectioneries as cookies, Japanese-style confectioneries as manju and yokan, candies, chewing-gum, custard pudding and chilled or ice confectioneries.

In preparing the food and drink according to the present invention, such saccharides, flavors, fruit juice, food additives, stabilizers and the like, as used in recipes of general foods and drinks, can be added appropriately. Preparation of the food and drink can be carried out referring to a preparing method publicly known in the art. The food and drink according to the present invention can take various forms, and the food and drink according to the present invention may be prepared based on a publicly known preparing method for pharmaceuticals. In this case, the carriers or the formulating additives as described hereinabove in the section for the preparation of the composition according to the present invention can be used for preparation, more specifically, the carriers or the formulating additives described hereinabove in the section for oral intake forms can be used for preparation. Further, by combining with other components or other functional foods, exerting a function other than the function according to the present invention, a multi-function food and drink can be prepared.

On the occasion of administration or intake of the composition and the food and drink according to the present invention, an administration or intake amount of the active ingredient according to the present invention may be determined appropriately reflecting the subject, the age and the body weight of the subject, the conditions of intestinal microflora, the administration time, the formulation form, the food form, the administrating method, the drug combination, *etc*. For example, by oral administration or oral intake of the active ingredient according to the present invention, based on the weight of 1-kestose, an intake dose is preferably in the range of 0.01 to 50 g per day per adult, more preferably 0.1 to 30 g and further preferably 1 to 20 g. The dose may be divided into 1 to several unit administration doses for (or unit doses intake daily). As described above, the intake dose of 1-ketose is preferably no more than 50 g. Considering wide difference among individuals in a laxative effect, however, the dose is more preferably no more than 30 g. Since there is an individual difference in a laxative effect, the dose is not restricted. Since the above administration amount or the intake amount is calculated as the administration amount or the intake amount of the active ingredient per day per adult with the body weight of 60 kg assuming the body weight of an adult at 60 kg, an optimized administration or intake amount can be obtained as required by a recalculation reflecting the body weight of the subject for administration or intake.

The composition according to the present invention includes 1-kestose as the active ingredient preferably at least 1% by weight based on the total weight of the composition, and more preferably includes 35% by weight or more.

In case of the food and drink according to the present invention, the amount of the added 1-kestose is preferably at least 1% by weight base on the total weight of the food and drink, in a more preferable aspect of the food and drink the amount is 1 to 90% by weight, and further preferably 1 to 50% by weight.

As described above, in addition to 1-kestose, viable cells of lactic acid bacteria may be added for use according to the present invention. In this case, if viable cells of lactic acid bacteria are in a freeze-dried cell form, the amount added in the composition according to the present invention is preferably equivalent to the intake amount in the range of 0.01 to 10 g per day per adult, and more preferably in the range of 0.1 to 3 g. Alternatively, if viable cells of lactic acid bacteria are in a freeze-dried cell form, the added amount of the same in the composition according to the present invention is preferably 0.01% by weight or more, and more preferably 0.1% by weight or more. Further, in case of the food and drink according to the present invention, the added amount of the same is preferably 0.001% by weight or more, more preferably 0.01% by weight or more.

### EXAMPLES

The present invention is further illustrated by the following Examples that are not intended as a limitation of the scope of the invention.

### Example 1: Growth effect on lactic acid bacteria in human intestine by intake of 1-kestose

A sample composition containing 99.4% of 1-kestose, 0.15% of acesulfame potassium, 0.32% of sucralose and 0.13% of Sweetness Enhancer ME-2208T (Trade Name) (Firmenich SA) was used (all by % by weight).
In a test, each 10 g per day of the sample composition was fed orally for 2 months to 7 male and female test subjects with mild diabetes in the age range of 20 to 70 years. Samples of the feces of the respective subjects were collected just before the start of intake (month 0), 2 months after the start of intake (month 2) and 1 month after the end of intake (month 3), and the lactic acid bacteria counts in the samples were quantitatively determined by the method of Mitsuoka (Mitsuoka Tomotari "Chounaikin no Sekai (The World of Intestinal Bacteria)" Sobunsha Co., Ltd., Tokyo, p. 53-65). Similarly, the *Bifidobacterium* counts in the samples were quantitatively determined.

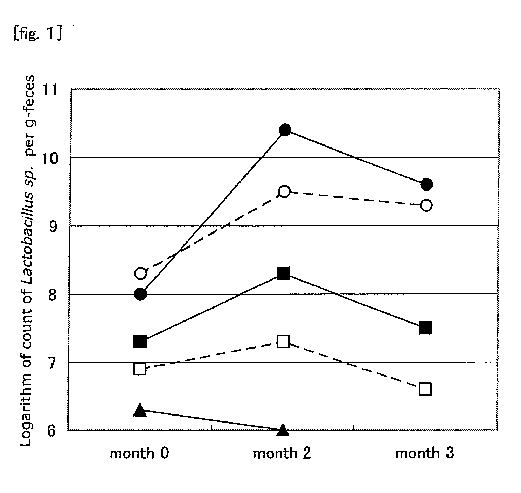

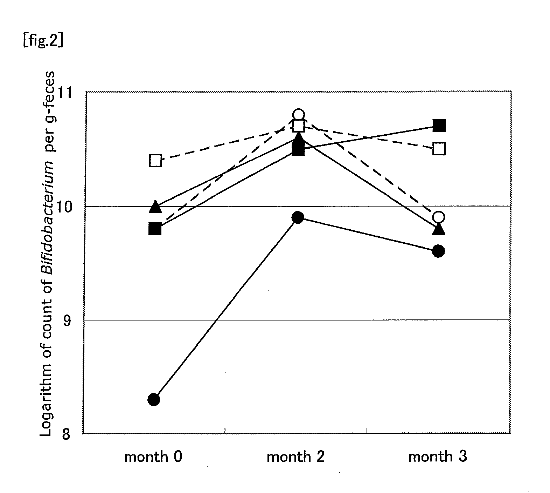

The results are shown in Figure 1 and Figure 2. By 5 out of the subjects 7, lactic acid bacteria belonging to genus *Lactobacillus* were detected at 10⁶ CFU/g-feces or higher, and the changes of the lactic acid bacteria count and the *Bifidobacterium* count by the intake of 1-kestose with respect to the 5 subjects are shown in the Figures.

As the result, by 4 out of the 5 subjects, the count of *Lactobacillus sp.* was increased by intake of 1-kestose. The mean value of the bacteria counts was increased about 10-fold from 10^{6.1} CFU/g-feces before the intake to 10^{7.1} CFU/g-feces after the intake. The increased lactic acid bacteria were identified by analysis of 16SrRNA sequence to find *Lactobacillus mucosae, Lactobacillus salivarius, Lactobacillus acidophilus, Lactobacillus lactis, Lactobacillus plantarum, Lactobacillus pentosus, Lactobacillus arizonensis, etc.* By all of 5 subjects, the *Bifidobacterium* count was increased, and the mean value was increased about 7-fold from 10^{9.7} CFU/g-feces before the intake to 10^{10.5} CFU/g-feces after the intake.

### Example 2:

As a sample composition, Meioligo P (Trade Name) (Meiji Seika Kaisha), which was a composition containing 35% of 1-kestose, 55% of nystose and 10% of fructo-furanosyl nystose, was used.
In a test, each 3 g per day of the sample composition (1.05 g based on the weight of 1-kestose) was fed orally for 2 months to 7 healthy female test subjects in the age range of 19 to 41 years. Samples of the feces of the respective subjects were then collected just before the start of intake (week 0), 2 weeks after the start of intake (week 2) and 2 weeks after the end of intake (week 4), and the lactic acid bacteria count and the *Bifidobacterium* count in the samples were quantitatively determined similarly to Example 1.

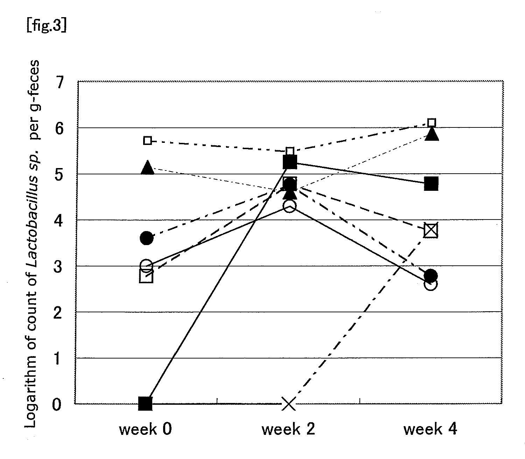

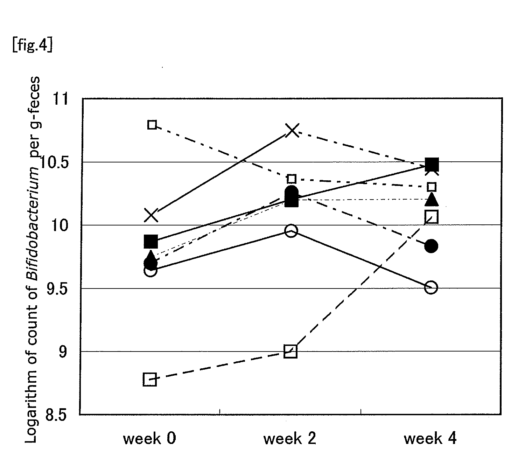

The results are shown in Figure 3 and Figure 4. The changes of the lactic acid bacteria count and the *Bifidobacterium* count in the feces by the intake of 1-kestose with respect to the 7 subjects are shown in the Figures.

As the result, by 4 out of 7 subjects, the count of *Lactobacillus sp.* was increased by intake of 1-kestose. The mean value of the bacteria counts in the feces was increased about 20-fold from 10^{2.9} CFU/g before the intake to 10^{4.2} CFU/g after the intake (a value less than the detection limit was put 0). By 3 subjects there was no increase, however there was no large decrease either. By all of 4 subjects with increase, the increase of 10-fold or higher was recognized. The increased lactic acid bacteria were identified by analysis of 16SrRNA sequence to find *Lactobacillus rhamnosus, Lactobacillus plantarum, Lactobacillus brevis, Lactobacillus caseii, Lactobacillus paracasei, etc.,* and, further, the increase of *Enterococcus faecium* and *Pediococcus pentosaceus* was observed. Further, by 6 out of the 7 subjects, the *Bifidobacterium* count was increased, and the mean value was increased about 2-fold from 10^{9.8} CFU/g before the intake to 10^{10.1} CFU/g after the intake.

## Claims

1. A composition for improving intestinal microflora comprising 1-kestose as an active ingredient.

2. The composition according to claim 1, wherein 1-kestose is provided in a range of 0.01 to 50 g per day per adult as the amount of 1-kestose.

3. The composition according to claim 1 or 2, comprising 1-kestose in at least 1% by weight.

4. The composition according to any one of claims 1 to 3, further comprising viable cells of lactic acid bacteria.

5. The composition according to any one of claims 1 to 4, wherein the improvement of intestinal microflora is caused by simultaneous proliferation of *bifidobacterium* and lactic acid bacteria in the intestine.

6. The composition according to any one of claims 1 to 5, which is provided in a form of a food and drink.

7. The composition according to any one of claims 1 to 5, which is provided in a form of a medicament.

8. A lactic acid bacteria proliferating agent for proliferating *Bifidobacterium* and lactic acid bacteria simultaneously, comprising 1-kestose as an active ingredient.

9. The proliferating agent according to claim 8, further comprising viable cells of lactic acid bacteria.

10. A food and drink comprising the lactic acid bacteria proliferating agent according to claim 8 or 9.

11. A food and drink comprising 1-kestose in an amount provided in a range of 0.01 to 50 g per day per adult, as well as viable cells of lactic acid bacteria.

12. The food and drink according to claim 11, comprising 1-kestose in at least 1% by weight.

13. The food and drink according to claim 11 or 12, which is used for improvement of intestinal microflora.

14. The food and drink according to claim 11 or 12, which is labeled with a function of improving intestinal microflora.

15. A method for improving intestinal microflora of mammals, comprising administering an effective amount of 1-kestose to mammals or allowing mammals to ingest an effective amount of 1-kestose.

16. The method according to claim 15, wherein an administered or ingested dose of 1-kestose is in a range of 0.01 to 50 g per day per adult as the amount of 1-kestose.

17. The method according to claim 15 or 16, wherein viable cells of lactic acid bacteria are administered or ingested together with 1-kestose.

18. The method according to any one of claims 15 to 17, comprising administering or ingesting viable cells of lactic acid bacteria prior to or simultaneously with 1-kestose so that a count of intestinal lactic acid bacteria in humans is 10⁶ CFU/g or more as measured in the feces.

19. The method according to any one of claims 15 to 18, wherein intestinal microflora is improved by simultaneous proliferation of *Bifidobacterium* and lactic acid bacteria in the intestine.

20. The method according to any one of claims 15 to 19, wherein 1-kestose and, optionally, viable cells of lactic acid bacteria are in-taken in a form of a food and drink.

21. Use of 1-kestose in the manufacture of a composition for improving intestinal microflora.

22. The use according to claim 21, wherein 1-kestose is provided in a range of 0.01 to 50 g per day per adult.

23. The use according to claim 21 or 22, wherein viable cells of lactic acid bacteria are further utilized.

24. The use according to claim 23, wherein viable cells of lactic acid bacteria are optionally further utilized so that a count of intestinal lactic acid bacteria is 10⁶ CFU/g or more as measured in the feces.

25. The use according to any one of claims 21 to 24, wherein intestinal microflora is improved by simultaneous proliferation of *Bifidobacterium* and lactic acid bacteria in the intestine.

26. The use according to any one of claims 21 to 25, wherein the composition for improving intestinal microflora is in a form of a food and drink.
